# EUROPEAN PATENT APPLICATION

(11) **EP 1 952 827 A1**
(43) Date of publication of application: **06.08.2008**
(21) Application number: 08150485.4
(22) Date of filing: 12.03.2003
(51) Int. Cl.: A61L 9/03, A61L 9/02, B60H 3/00

(54) **Electric devise for scenting the interior of vehicles**

(30) Priority: 15.03.2002 IT PI20020015
(62) Divisional of application: 03715345.9
(71) Applicant: EUREKA S.N.C, 52100 Arezzo (IT); Sarong, S.P.A., 42046 Reggiolo (IT)
(72) Inventor: Fantoni, Adriano, 52100, Arezzo (IT); Magrini, Giancarlo, 52100, Arezzo (IT); Borri, Gianmatteo, 52100, Arezzo (IT)
(74) Representative: Colò, Chiara

(57) **Abstract**

A device for scenting the interior of vehicles comprises:
- means to scent the interior of a vehicle with a perfumed capsule sending out vapours after its heating at a prefixed temperature, said capsule being made of plastic material holding a perfume and having a surface provided with cells permitting the perfume to exude;
- means to heat said capsule by an electric or electronic component (14) able to produce heat, said electric or electronic component (14) being connected to a circuit powered by the circuit of the vehicle;
- means to turn on and off the device, with a switch (4) installed in the circuit.

## Description

The present invention relates to the technical sector of the production of accessories for cars and particularly of devices used to scent the interior of a car or other vehicles.

Several car scenting devices are nowadays used, principally constituted by items of different shape, made of a material filled with perfume, so that, once partially or totally removed from their protective film, they diffuse perfume in the air. Such a kind of device has however the drawback that it does not regulate the appropriate gradation of perfume and it keeps on emitting perfume in any moment, when the vehicle is on as well as off, and the user cannot do anything to stop this process.

Other scenting devices also exist, consisting of a liquid perfume receptacle holding a sponge with its upper part placed in front of an accessory that is applied on the heaters, so that when the air comes into the vehicle, it makes the perfume diffused. But also this product has relevant drawbacks: first of all, the high cost of the refill and its wear by evaporation, in any case, without considering that accidental falls might spill the liquid and imply relative negative consequences.

The present invention principally aims at supplying a new device able to diffuse a perfect perfume in the interior, adjustable according to the user's need, having the characteristics described in the separate claims. Other characteristics of this invention are the object of dependent claims.

The advantages resulting from the present invention essentially consist of the fact that it is possible to make the interior of a vehicle scented in any moment, but only if desired; that the device is clean and small, easy to use and difficult to wear; that the cost of the refills of perfume is reduced; that its working system allows to make the interior scented, using only a small amount of perfume; that it is powered by the 12V/24V circuit of the same vehicle. These and further advantages of the present invention can be better understood by every expert in this field by reading the following description and referring to the enclosed drawings.

Reduced to its essential structure and with reference to the figures of the enclosed drawings, a device for scenting the interior of vehicles, according to the invention, comprises:
- means to scent the interior of a vehicle, with a perfumed capsule sending out vapours only after its heating at a prefixed temperature;
- means to heat said capsule by an electric or electronic component able to produce heat, connected to a circuit powered by the circuit of the vehicle it is connected to;
- means to turn on and off the device, with a switch installed in the circuit, possibly connected to a luminous led informing the user about the on/off status of the same device.

Conveniently, the capsule is inserted into the device from the outside by a suitable opening (6) and is placed on a support (16) inside the device near the heating component, so that when said component heats, the warmth rapidly transfers to the capsule too.

Conveniently, the scented capsule is made of plastic material holding perfumed gel and its upper surface is made of silicone material with cells permitting the perfume to exude.

Conveniently, in a practical example, the component able to produce heat consists of a resistance (14) connected to the electric circuit.

Conveniently, the device can be either integrated into the vehicle or separated as an accessory to be connected to the car electric circuit when used.

Conveniently, in one part (1) the device takes a pin shape equipped with a rounded tip (2), a prod (7) and two flaps (3), so that it is fitted into the socket of the cigarette lighter in order to take current.

Conveniently, this device works with a 12V/24V current.

Conveniently, this device includes a safety fuse (8) that is applied to the circuit in order to protect it from possible overcharges. Said fuse can be removed and replaced in case it is broken.

Conveniently, this device has a series of holes on one or more parts of its surface, permitting the perfume to come out better.

This is the way this device works:
Fitting the device into the socket of the car cigarette lighter, it turns on, sets the led on, and starts the heat of the resistance or other electric/electronic component able to produce heat, provided that the switch is on. Once the selected temperature has been reached, the process of diffusing the perfume held into the scented capsule starts and ends only when the device is turned off by its switch or by its removing from the socket of the cigarette lighter.
In practice, the manufacturing details may, however, vary as regards shape, size, position of elements, and type of materials used, but still remain within the range of the idea proposed as a solution and, consequently, within the limits of the protection granted by this patent for invention. The enclosed drawings are given as practical examples of the invention, but are not to be considered restrictive.

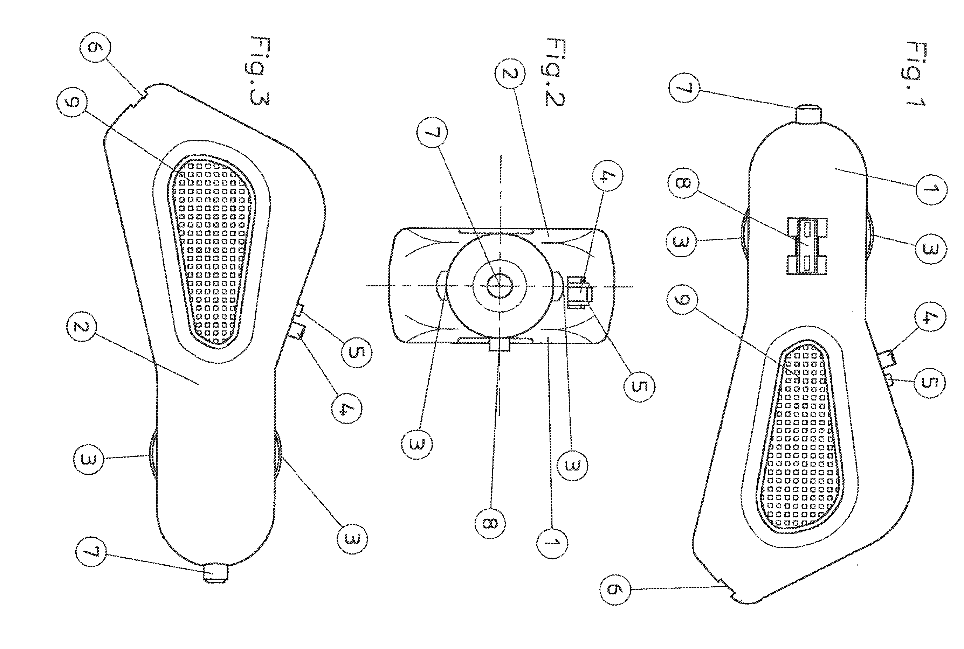

Figure 1 shows an external view of the device with the plastic body having an end part (1) to be fitted into the socket of the car cigarette lighter, so that by means of its tip (7) and its flaps (3) it powers the internal circuit. The same figure also shows the led (5), the switch (4) used to turn the device on/off, the opening (6) inside which the scented capsule is inserted and a surface with holes (9) where perfume exudes.
Figure 2 shows the front view of the same device as Figure 1.
Figure 3 shows the opposite side of the same device as Figure 1.

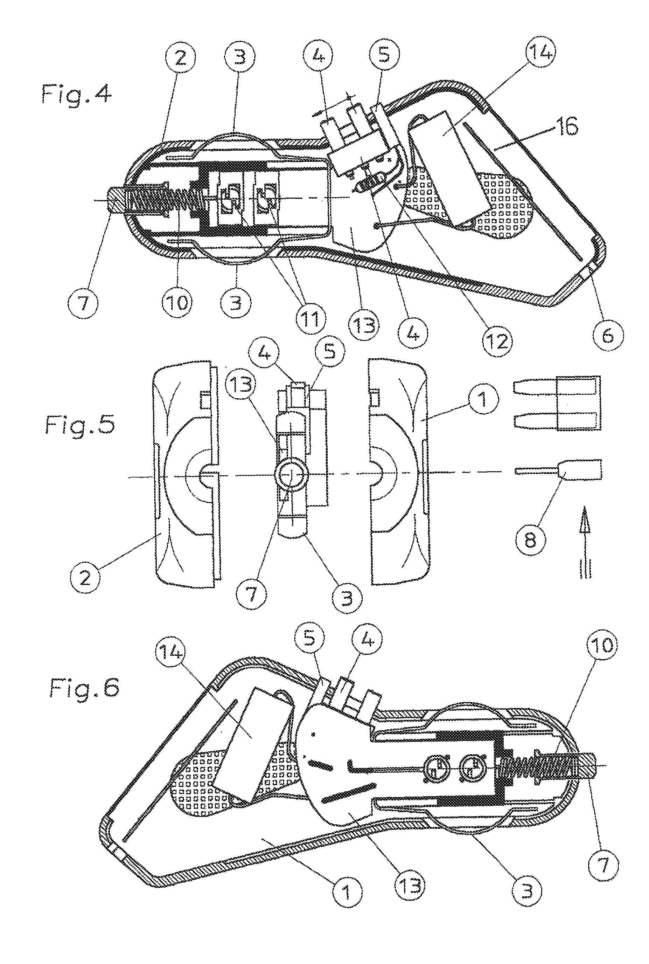

Figure 4 shows the inside of the device, with an end rounded part (2) to be fitted into the socket of the car cigarette lighter, the flaps (3) and the tip (7) joined to a spring (10) connected to the circuit comprising two holders (11) inside which the wires of the fuse (8) enter. The circuit is connected to a switch (4) joined to a led (5) and to a power resistance (12). The circuit is also connected to a resistance (14) having the function to heat the perfume placed over the support (16) fitting it through the opening (6).
Figure 5 shows the front exploded view of the device.
Figure 6 shows the opposite inside of the device.

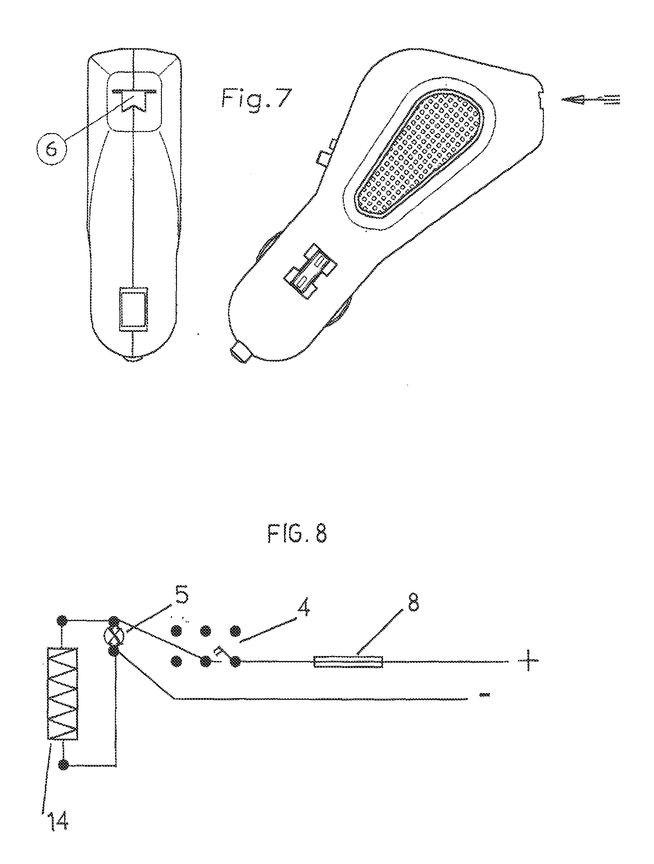

Figure 7 shows the final device, highlighting the opening (6) through which the scented capsule is inserted.
Figure 8 shows the electric circuit of the device, with the resistance (14), the switch (4), the led (5) and the protective fuse (8).

## Claims

1. Device for scenting the interior of vehicles, comprising:
- means to scent the interior of a vehicle with a perfumed capsule sending out vapours after its heating at a prefixed temperature, said capsule being made of plastic material holding a perfume and having a surface provided with cells permitting the perfume to exude;
- means to heat said capsule by an electric or electronic component (14) able to produce heat, said electric or electronic component (14) being connected to a circuit powered by the circuit of the vehicle;
- means to turn on and off the device, with a switch (4) installed in the circuit.

2. Device according to claim 1, wherein the circuit included in said device is connected to a luminous led (5) informing the user about the on/off status of the device.

3. Device according to claim 1, and having an opening (6) through which the capsule can be inserted into the device from the outside and a support (16) on which the capsule can be placed, the support (16) being located inside the device near said electric or electronic component (14), so that when said electric or electronic component (14) heats, the warmth rapidly transfers to the capsule too.

4. Device according to claim 1, wherein the electric or electronic component able to produce heat consists of a resistance (14) connected to the circuit.

5. Device according to claim 1, wherein said device can be either integrated into the vehicle or separated as an accessory to be connected to the car electric circuit when used.

6. Device according to claim 1, and further comprising one part (1) having the shape of a pin equipped with a rounded tip (2), a prod (7) and two flaps (3), so that it can be fitted into the socket of a cigarette lighter in order to take current.

7. Device according to claim 1, wherein said device works with a 12V/24V current.

8. Device according to claim 1, and further comprising a safety fuse (8), which can be removed and replaced, applied to the circuit in order to protect it from possible overcharges.

9. Device according to claim 1, and further comprising a series of holes (9) on one or more parts of its surface, permitting the perfume to come out better.
